(19) Europäisches Patentamt
European Patent Office
Office européen des brevets

(11) Veröffentlichungsnummer: **0 158 955**
**A2**

(12) # EUROPÄISCHE PATENTANMELDUNG

(21) Anmeldenummer: 85104251.5

(22) Anmeldetag: 09.04.85

(51) Int. Cl.⁴: C 07 D 211/60
C 07 D 401/04, C 07 D 413/04
C 07 D 417/04, C 07 D 409/04
C 07 D 407/04, A 61 K 31/445

(30) Priorität: 19.04.84 DE 3414802

(43) Veröffentlichungstag der Anmeldung: 23.10.85 Patentblatt 85/43

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Rosentreter, Ulrich, Dr.
Kondorweg 23
D-5600 Wuppertal 1(DE)

(72) Erfinder: Thomas, Günther, Dr.
Claudiusweg 9
D-5600 Wuppertal 1(DE)

(72) Erfinder: Knorr, Andreas, Dr.
Pahlkestrasse 15
D-5600 Wuppertal 1(DE)

(54) **Hexahydropyridine, Verfahren zu ihrer Herstellung und ihre Verwendung als Arzneimittel.**

(57) Die vorliegende Erfindung betrifft neue Hexahydropyridine der allgemeinen Formel I

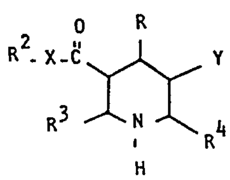

(I)

in welcher R, $R^1$, $R^2$, $R^3$, $R^4$, Y und X die in der Beschreibung angegebene Bedeutung haben, ein Verfahren zu ihrer Herstellung sowie ihre Verwendung als Arzneimittel, insbesondere als kreislaufbeeinflussende Mittel.

EP 0 158 955 A2

Croydon Printing Company Ltd

BAYER AKTIENGESELLSCHAFT 5090 Leverkusen, Bayerwerk

Konzernverwaltung RP Ks/by-c
Patentabteilung Ia(Pha)

# Hexahydropyridine, Verfahren zu ihrer Herstellung und ihre Verwendung als Arzneimittel

Die vorliegende Erfindung betrifft neue Hexahydropyridine, ein Verfahren zu ihrer Herstellung sowie ihre Verwendung als Arzneimittel, insbesondere als kreislaufbeeinflussende Mittel.

Die vorliegende Erfindung betrifft neue Hexahydropyridine der allgemeinen Formel (I),

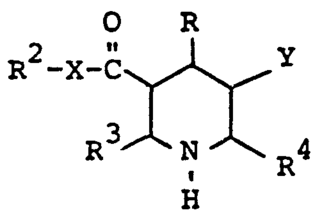

(I)

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest gegebenenfalls 1 bis 2 gleiche oder verschiedene Substituenten aus der Gruppe Phenyl, Alkyl, Alkoxy, Alkylen, Dioxyalkylen, Halogen, Trifluormethyl, Polyfluoralkoxy, Nitro oder Cyano enthält,

Y für Cyano oder den Rest $CO-X^1-R^1$ steht,

$R^1$ und $R^2$ gleich oder verschieden sind und jeweils für einen geradkettigen, verzweigten oder cyclischen, gesättigten oder ungesättigten Kohlenwasserstoffrest stehen, der gegebenenfalls durch ein Sauerstoff- oder Schwefelatom in der Kette unterbrochen ist, und/oder der gegebenenfalls substituiert ist durch Halogen, Cyano, Hydroxy, Pyridyl, Phenyl, Phenoxy oder Phenylthio, wobei die Phenylgruppen ihrerseits substituiert sein können durch Halogen, Nitro, Cyano, Dialkylamino, Alkoxy, Alkyl, Trifluormethyl oder Polyfluoralkoxy, oder durch eine Aminogruppe, die durch zwei gleiche oder verschiedene Substituenten aus der Gruppe Alkyl, Aryl oder Aralkyl substituiert ist,

$R^3$ und $R^4$ gleich oder verschieden sind und jeweils für Wasserstoff, einen geradkettigen oder verzweigten Alkylrest, einen Arylrest, einen Aralkylrest oder für einen Carboxyalkylrest stehen, und

X und $X^1$ gleich oder verschieden sind und jeweils für eine Einfachbindung, ein Sauerstoffatom, ein Schwefelatom oder für die Gruppe -NH stehen,

sowie ihre physiologisch unbedenklichen Salze.

Es wurde gefunden, daß man die erfindungsgemäßen Verbindungen der Formel (I) erhält, wenn man Dihydropyridinverbindungen der Formel (II),

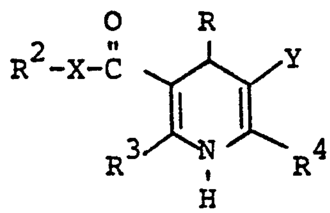

(II)

in welcher

$R, R^1, R^2, R^3, R^4$, Y und X die oben angegebene Bedeutung haben,

in einem stark sauren Medium durch geeignete Hydriddonoren reduziert.

Die erfindungsgemäßen 1,2,3,4-Tetrahydropyridinderivate besitzen wertvolle phamakologische Eigenschaften. Aufgrund ihrer kreislaufbeeinflussenden Wirkung können sie als antihypertensive Mittel, als periphere und cerebrale Vasodilatatoren sowie als Coronartherapeutika Verwendung finden und sind somit als Bereicherung der Pharmazie anzusehen.

Die Darstellung der erfindungsgemäßen Verbindungen kann durch folgendes Formelschema wiedergegeben werden, wobei der 2,6-Dimethyl-4-(2-nitrophenyl)-hexahydropyridin-3,5-dicarbonsäuredimethylester als Beispiel gewählt sei:

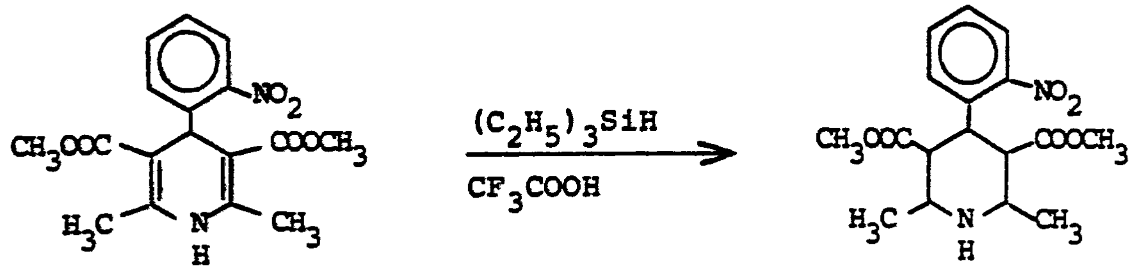


Nach dem erfindungsgemäßen Verfahren wird die Reduktion der eingesetzten 1,4-Dihydropyridine (II) in einem stark sauren Reaktionsmedium durchgeführt.

Als stark saure Reaktionsmedien kommen vorzugsweise in Frage:
Trifluoressigsäure, Mischungen von Trifluoressigsäure mit Essigsäure, Dichlormethan, Chloroform, Benzol, Toluol, 1,2-Dichlorethan, Dioxan, Tetrahydrofuran, Nitromethan und Acetonitril, Mischungen von Schwefelsäure mit Eisessig, Lösungen von Methansulfonsäure oder p-Toluolsulfonsäure in Dichlormethan, Chloroform, Benzol, Toluol, 1,2-Dichlorethan, Dioxan, Tetrahydrofuran, Nitromethan und Acetonitril, sowie Mischungen dieser sauren Reaktionsmedien.

Die Reduktion erfolgt durch Zugabe von 2 bis 6 Äquivalenten eines Hydriddonors wie z.B. Triethylsilan,

Triphenylsilan, Tributylzinnhydrid, Natriumcyanoborhydrid, 1,4-Dihydronaphthalin oder 1,4-Cyclohexadien.

Die Reaktionstemperatur kann breit variiert werden, vorzugsweise arbeitet man jedoch zwischen 0°C und 60°C.

In den Formeln (I) und (II) stehen vorzugsweise

R für Phenyl, Naphthyl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl, wobei die genannten Carbocyclen jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxamethylen, Halogen, Trifluormethyl, Trifluormethoxy, Nitro, Difluormethoxy, Tetrafluorethoxy, Cyano substituiert sein können,

$R^1$ und $R^2$ jeweils für einen geradkettigen, verzweigten oder cyclischen, gesättigten oder ungesättigten Kohlenwasserstoffrest mit bis zu 20 Kohlenstoffatomen, der gegebenenfalls durch ein Sauerstoff oder Schwefel in der Kette unterbrochen ist und/ oder der gegebenenfalls substituiert ist durch

Halogen, Cyano, Hydroxy, α-, ß- oder γ-Pyridyl, Phenyl, Phenoxy oder Phenylthio, wobei die Phenylgruppen substituiert sein können durch Halogen, durch Cyano, Nitro, Dialkylamino mit 1 bis 4 Kohlenstoffatomen je Alkylkette, Alkoxy mit 1 bis 4 Kohlenstoffatomen, Alkyl mit 1 bis 4 Kohlenstoffatomen, Trifluormethyl oder Trifluormethoxy, oder durch eine Aminogruppe, die ihrerseits durch zwei gleiche oder verschiedene Substituenten aus der Gruppe Alkyl mit 1 bis 4 Kohlenstoffatomen, Phenyl oder Benzyl substituiert ist,

Y für Cyano oder den Rest $CO\text{-}X^1\text{-}R^1$,

$R^3$ und $R^4$ die gleich oder verschieden sind, jeweils für Wasserstoff, einen geradkettigen oder verzweigten Alkylrest mit bis zu 4 Kohlenstoffatomen, einen Phenylrest oder einen Carboxyalkylrest mit bis zu 4 Kohlenstoffatomen,

X und $X^1$ für eine Einfachbindung oder ein Sauerstoff.

Von besonderem Interesse sind Verbindungen der Formel (I) in welcher

R für Phenyl oder Pyridyl steht, wobei der Phenylrest gegebenenfalls durch ein oder zwei gleiche oder verschiedene Substituenten aus der Gruppe Nitro, Fluor, Chlor, Trifluormethyl, Trifluormethoxy, Cyano,

Alkyl mit 1 bis 4 C-Atomen, Alkoxy mit 1 bis 4 C-Atomen, Dioxymethylen substituiert ist,

$R^1$ und $R^2$ gleich oder verschieden sind und jeweils für einen geradkettigen oder verzweigten oder cyclischen gesättigten Kohlenwasserstoffrest mit bis zu 12 C-Atomen stehen, der gegebenenfalls durch ein Sauerstoff in der Kette unterbrochen ist und/oder der gegebenenfalls substituiert ist durch Fluor, Chlor, Cyano oder Hydroxy,

Y für Cyano oder den Rest $CO-X^1-R^1$ steht,

$R^3$ und $R^4$ jeweils gleich oder verschieden sind und für Alkyl mit 1 bis 4 Kohlenstoffatomen stehen und

X und $X^1$ gleich oder verschieden sind und jeweils für eine Einfachbindung oder ein Sauerstoff stehen,

sowie ihre physiologisch unbedenklichen Säureadditionssalze.

Die als Ausgangsstoffe verwendeten 1,4-Dihydropyridinderivate der allgemeinen Formel (II) sind literaturbekannt oder können nach literaturbekannten Methoden hergestellt werden (vgl. z.B. U. Eisner und J. Kuthan, Chem. Rev. 72, 1 (1972); E. Wehinger, F. Bossert, G. Franckowiak und H. Meyer, Deutsche Offenlegungsschrift 26 58 804, Publ. Date: 6.7.1978).

Je nach Art der Reste R bis $R^4$, X und Y enthalten die erfindungsgemäßen Verbindungen mindestens drei Asymmetriezentren und können daher in mehreren stereoisomeren Formen auftreten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Außer den unten angeführten Herstellungsbeispielen seien folgende erfindungsgemäße Wirkstoffe genannt:
t-2,t-6-Dimethyl-t-4-(3-nitrophenyl)-hexahydropyridin-r-3,c-5-dicarbonsäurediethylester,
t-2,t-6-Dimethyl-t-4-(3-nitrophenyl)-hexahydropyridin-r-3,c-5-dicarbonsäuredipropylester,
t-2,t-6-Dimethyl-t-4-(3-nitrophenyl)-hexahydropyridin-r-3,c-5-dicarbonsäure-di-(2-methoxyethyl)ester,
t-2,t-6-Diethyl-t-4-(3-nitrophenyl)-hexahydropyridin-r-3,c-5-dicarbonsäuredimethylester,
t-2,t-6-Dimethyl-t-4-(3-nitrophenyl)-hexahydropyridin-r-3-carbonsäureisopropylester-c-5-carbonsäuremethylester,
t-2,t-6-Dimethyl-t-4-(3-nitrophenyl)-hexahydropyridin-r-3-carbonsäureethylester-c-5-carbonsäuremethylester,
t-2,t-6-Dimethyl-t-4-(3-nitrophenyl)-hexahydropyridin-r-3-carbonsäureisopropylester-5-carbonsäure(2-methoxyethyl)ester

Die neuen Verbindungen haben ein breites und vielseitiges pharmakologisches Wirkungsspektrum.

Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten Muskulatur des Magens, Darmtraktes, des Urogenitaltraktes und des Respirationssystems deutlich werden.

Die erfindungsgemäßen Verbindungen eignen sich aufgrund dieser Eigenschaften besonders zur Prophylaxe und Therapie der akuten und chronischen ischämischen Herzkrankheiten im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln

und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß.

Beispiel 1

t-2,t-6-Dimethyl-t-4-(2-nitrophenyl)-hexahydropyridin-r-3,c-5-dicarbonsäuredimethylester

10 g (29 mmol) 2,6-Dimethyl-4-(2-nitrophenyl)-1,4-dihydropyridin-3,5-dicarbonsäuredimethylester werden in 100 ml Trifluoressigsäure gelöst und mit 14 ml (87 mmol) Triethylsilan versetzt. Anschließend wird 3 Stunden bei 50°C gerührt. Dann wird mit viel Wasser verdünnt und 2 mal mit Essigester extrahiert. Die vereinigten Essigesterphasen werden 3 mal mit gesättigter Bicarbonatlösung gewaschen und mit Magnesiumsulfat getrocknet. Der nach dem Eindampfen erhaltene Rückstand kristallisiert aus Ether.
Ausbeute: 9,5 g (94 % der Theorie), Fp.: 142°C.

Beispiel 2

t-2,t-6-Dimethyl-t-4-(2-nitrophenyl)-hexahydropyridin-r-3,t-5-dicarbonsäuredimethylester

10 g (29 mmol) 2,6-Dimethyl-4-(2-nitrophenyl)-1,4-dihydropyridin-3,5-dicarbonsäuredimethylester werden in 100 ml Trifluoressigsäure gelöst und auf 0°C gekühlt. Dann werden 10,9 g (174 mmol) Natriumcyanoborhydrid portionsweise so zugegeben, daß die Temperatur +5°C nicht übersteigt. Nach beendeter Zugabe läßt man die

Reaktionsmischung langsam auf Raumtemperatur kommen. Der Ansatz wird über Nacht bei Raumtemperatur gerührt, dann mit 50 ml Wasser versetzt und 2 Stunden gerührt (HCN-Entwicklung). Nach Verdünnen mit viel Wasser wird 2 mal mit Essigester extrahiert und die vereinigten Essigesterphasen werden 3 mal mit gesättigter Bicarbonatlösung gewaschen. Nach Trocknen mit Magnesiumsulfat wird eingedampft und das so erhaltene, bereits kristalline Rohprodukt durch Säulenchromatographie gereinigt (Merck Kieselgel 60 0,04 bis 0,063 mm, Toluol/Essigester 1:1 Gemisch als Laufmittel). Nach Eindampfen der reinen Fraktionen erhält man 2,7 g festen Rückstand, der aus Ether umkristallisiert wurde.
Ausbeute: 2,4 g (24 % der Theorie), Fp.: 137°C.

Beispiel 3

t-2,t-6-Dimethyl-t-4-(2-trifluormethylphenyl)-hexahydropyridin-r-3,t-5-dicarbonsäuredimethylester

Die Synthese dieser Verbindung aus 2,6-Dimethyl-4-(2-trifluormethylphenyl)-1,4-dihydropyridin-3,5-dicarbonsäuredimethylester verlief analog zu Beispiel 2.
Ausbeute: 2 g (20 % der Theorie), Fp.: 96-98°C.

Die in Tabelle 1 angeführten Verbindungen lassen sich analog zu Beispiel 1 herstellen

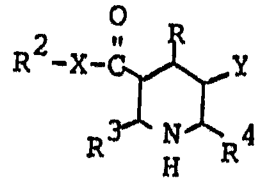

Tabelle 1

| Nr. | R | $R^2$ | $R^3$ | X | $R^4$ | Y | Schmelzpunkt (°C) | Ausbeute in % der Theorie |
|---|---|---|---|---|---|---|---|---|
| 4 | 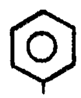 | $CH_3$ | $CH_3$ | O | $CH_3$ | $COOCH_3$ | 122-124 | 55 |
| 5 | 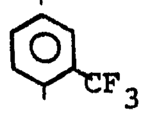 | $CH_3$ | $CH_3$ | O | $CH_3$ | $COOCH_3$ | 70 | 56 |
| 6 | 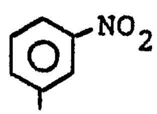 | $C_2H_5$ | $CH_3$ | O | $COOC_2H_5$ | $COOC_2H_5$ | 110 | 72 |
| 7 | 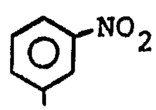 | $CH_3$ | $CH_3$ | Einfach-bindung | $CH_3$ | $COCH_3$ | 138 | 60 |
| 8 | 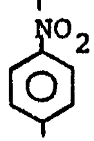 | $C_2H_5$ | $CH_3$ | O | $CH_3$ | $COOC_2H_5$ | 114-117 | 69 |
| 9 | 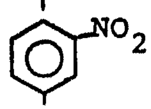 | $CH_3$ | $CH_3$ | O | $CH_3$ | CN | 112 | 89 |
| 10 | 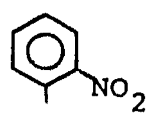 | $C_2H_5$ | $CH_3$ | O | $CH_3$ | $COOC_2H_5$ | 65-68 | 64 |

Patentansprüche

1. Hexahydropyridine der allgemeinen Formel (I)

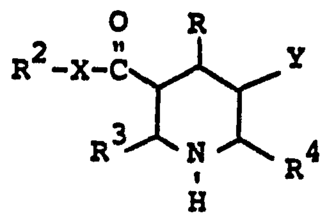

(I)

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest gegebenenfalls 1 bis 2 gleiche oder verschiedene Substituenten aus der Gruppe Phenyl, Alkyl, Alkoxy, Alkylen, Dioxyalkylen, Halogen, Trifluormethyl, Polyfluoralkoxy, Nitro oder Cyano enthält,

Y für Cyano oder den Rest $CO-X^1-R^1$ steht,

$R^1$ und $R^2$ gleich oder verschieden sind und jeweils für einen geradkettigen, verzweigten oder cyclischen, gesättigten oder ungesättigten Kohlenwasserstoffrest stehen, der gegebenenfalls durch ein Sauerstoff- oder Schwefelatom

in der Kette unterbrochen ist, und/oder der gegebenenfalls substituiert ist durch Halogen, Cyano, Hydroxy, Pyridyl, Phenyl, Phenoxy oder Phenylthio, wobei die Phenylgruppen ihrerseits substituiert sein können durch Halogen, Nitro, Cyano, Dialkylamino, Alkoxy, Alkyl, Trifluormethyl oder Polyfluoralkoxy, oder durch eine Aminogruppe, die durch zwei gleiche oder verschiedene Substituenten aus der Gruppe Alkyl, Aryl oder Aralkyl substituiert ist,

$R^3$ und $R^4$ gleich oder verschieden sind und jeweils für Wasserstoff, einen geradkettigen oder verzweigten Alkylrest, einen Arylrest, einen Aralkylrest oder für einen Carboxyalkylrest stehen, und

X und $X^1$ gleich oder verschieden sind und jeweils für eine Einfachbindung, ein Sauerstoffatom, ein Schwefelatom oder für die Gruppe -NH stehen,

sowie ihre physiologisch unbedenklichen Salze.

2. Verbindungen der allgemeinen Formel (I) gemäß Anspruch 1, in welcher die folgenden Substituenten stehen

R für Phenyl, Naphthyl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl, wobei die genannten Carbocyclen jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxamethylen, Halogen, Trifluormethyl, Trifluormethoxy, Nitro, Difluormethoxy, Tetrafluorethoxy, Cyano substituiert sein können,

$R^1$ und $R^2$ jeweils für einen geradkettigen, verzweigten oder cyclischen, gesättigten oder ungesättigten Kohlenwasserstoffrest mit bis zu 20 Kohlenstoffatomen, der gegebenenfalls durch ein Sauerstoff oder Schwefel in der Kette unterbrochen ist und/ oder der gegebenenfalls substituiert ist durch Halogen, Cyano, Hydroxy, $\alpha$-, ß- oder $\gamma$-Pyridyl, Phenyl, Phenoxy oder Phenylthio, wobei die Phenylgruppen substituiert sein können durch Halogen, durch Cyano, Nitro, Dialkylamino mit 1 bis 4 Kohlenstoffatomen je Alkylkette, Alkoxy mit 1 bis 4 Kohlenstoffatomen, Alkyl mit 1 bis 4 Kohlenstoffatomen, Trifluormethyl oder Trifluormethoxy, oder

durch eine Aminogruppe, die ihrerseits durch zwei gleiche oder verschiedene Substituenten aus der Gruppe Alkyl mit 1 bis 4 Kohlenstoffatomen, Phenyl oder Benzyl substituiert ist,

Y für Cyano oder den Rest $CO-X^1-R^1$,

$R^3$ und $R^4$ die gleich oder verschieden sind, jeweils für Wasserstoff, einen geradkettigen oder verzweigten Alkylrest mit bis zu 4 Kohlenstoffatomen, einen Phenylrest oder einen Carboxyalkylrest mit bis zu 4 Kohlenstoffatomen,

X und $X^1$ für eine Einfachbindung oder ein Sauerstoff.

3. Verbindungen der allgemeinen Formel (I) gemäß Anspruch 1, in welcher

R für Phenyl oder Pyridyl steht, wobei der Phenylrest gegebenenfalls durch ein oder zwei gleiche oder verschiedene Substituenten aus der Gruppe Nitro, Fluor, Chlor, Trifluormethyl, Trifluormethoxy, Cyano, Alkyl mit 1 bis 4 C-Atomen, Alkoxy mit 1 bis 4 C-Atomen, Dioxymethylen substituiert ist,

$R^1$ und $R^2$ gleich oder verschieden sind und jeweils für einen geradkettigen oder verzweigten oder cyclischen gesättigten Kohlenwasserstoffrest

mit bis zu 12 C-Atomen stehen, der gegebenenfalls durch ein Sauerstoff in der Kette unterbrochen ist und/oder der gegebenenfalls substituiert ist durch Fluor, Chlor, Cyano oder Hydroxy,

Y für Cyano oder den Rest $CO\text{-}X^1\text{-}R^1$ steht,

$R^3$ und $R^4$ jeweils gleich oder verschieden sind und für Alkyl mit 1 bis 4 Kohlenstoffatomen stehen und

X und $X^1$ gleich oder verschieden sind und jeweils für eine Einfachbindung oder ein Sauerstoff stehen.

4. Verfahren zur Herstellung von Verbindungen der allgemeinen Formel (I) gemäß Anspruch 1, dadurch gekennzeichnet, daß man Dihydropyridinverbindungen der allgemeinen Formel (II)

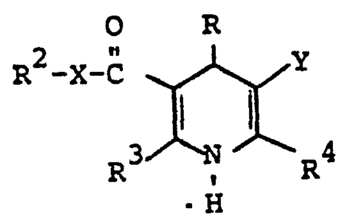

(II)

in welcher

$R, R^1, R^2, R^3, R^4$, Y und X die oben angegebene Bedeutung haben,

in einem stark sauren Medium durch geeignete Hydriddonoren reduziert.

5. Verfahren gemäß Anspruch 4, dadurch gekennzeichnet, daß man bei Reaktionstemperaturen zwischen 0°C und 60°C arbeitet.

6. Verfahren gemäß Anspruch 4, dadurch gekennzeichnet, daß man als saures Reaktionsmedium starke organische Säuren oder Mischungen aus anorganischen oder organischen Säuren mit inerten organischen Lösungsmitteln verwendet.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1 gegebenenfalls unter Verwendung von Hilfs- und Trägerstoffen in eine geeignete Applikationsform überführt.

9. Verwendung von Verbindungen der allgemeinen Formel (I) gemäß Anspruch 1 bei der Bekämpfung von Erkrankungen.

10. Verwendung von Verbindungen der allgemeinen Formel (I) gemäß Anspruch 1 bei der Bekämpfung von Kreislauferkrankungen.